(19) **Europäisches Patentamt**

**European Patent Office**

**Office européen des brevets**

(11) **EP 1 192 860 B1**

(12) **EUROPEAN PATENT SPECIFICATION**

(45) Date of publication and mention
of the grant of the patent:
**01.09.2004 Bulletin 2004/36**

(51) Int Cl.⁷: **A01N 47/44**, A01N 37/44,
A01N 35/02, A01N 33/12,
A01N 33/04, A61L 2/18,
G01N 21/63, G01N 21/33

(21) Application number: **01203447.6**

(22) Date of filing: **11.09.2001**

(54) **UVA-tracer containing disinfectant and method of determining the degree of dilution of disinfectants using UVA-tracers**

Disinfektionsmittel enthaltend einen UVA-Indikator und Verfahren zur Feststellung der Verdünnungsgrad mit Hilfe eines UVA-Indikators

Désinfectant conténant un traceur-UVA et méthode pour déterminer la dilution des désinfectants avec les traceurs-UVA

(84) Designated Contracting States:
**DE ES FR GB IT**

(30) Priority: **29.09.2000 DE 10048391**

(43) Date of publication of application:
**03.04.2002 Bulletin 2002/14**

(73) Proprietors:
• **Air Liquide Santé (International)**
**75341 Paris Cedex 07 (FR)**
Designated Contracting States:
**ES FR GB IT**
• **SCHÜLKE & MAYR GMBH**
**22851 Norderstedt (DE)**
Designated Contracting States:
**DE**

(72) Inventors:
• **Löwer, Bernd**
**22337 Hamburg (DE)**
• **Starp, Helmut**
**48351 Everswinkel (DE)**
• **Thiede, Joachim**
**22850 Nordestedt (DE)**
• **Buschmann, Norbert**
**48268 Greven (DE)**

(74) Representative: **Conan, Philippe Claude et al**
**L'Air Liquide SA,**
**75 Quai d'Orsay**
**75321 Paris Cedex 01 (FR)**

(56) References cited:
**EP-A- 0 320 086**     **EP-A- 0 680 694**
**WO-A-98/20094**      **GB-A- 2 338 242**

## Description

The present invention relates to compositions which comprise at least one microbicidal active ingredient and at least one UVA tracer. The invention further relates to a method of determining the degree of dilution of compositions which have a disinfecting action using UVA-tracers.

In daily disinfection practice, use is made of chemical disinfectants based on liquid preparations which are mostly offered on the market in the form of concentrates and then, for use, diluted with water to give a working solution. A disinfectant should be effective at as low as possible a concentration and be microbicidally effective as rapidly as possible over a broad activity spectrum. (Here, microbicidal action encompasses bacteriocidal, fungicidal, virucidal and/or sporicidal action.) Further requirements for an ideal disinfectant are adequate stability of the concentrate, good material compatibility and general safety for people, animals and the environment. Since no disinfectant active ingredient can combine all of these properties by itself, different active ingredients are frequently combined with one another in so-called combination preparations.

In particular, the requirement for disinfectants with good skin compatibility, low toxicity and good environmental compatibility has led to substances such as active chlorine and aldehydes being replaced by cationic surfactants to an increasing degree in recent years, the quaternary ammonium compounds (quats) being preferred cationic surfactants. The market share of surface disinfectants containing quats in Germany is currently more than 80%.

The effect of all disinfectants is heavily dependent on the active ingredient concentration. Depending on the field of use and contact time, use is made, for the disinfection, of dilutions in water which have a content of from 0.1 g to 2 g of active ingredient per litre of the working solution. The majority of the active ingredients only have microbicidal effectiveness in a correspondingly high concentration, and in an approximately 5 to 10 times lower concentration have only a growth-inhibiting effect on microbes. Accordingly, as high an active ingredient concentration as possible in the working solution is desired. However, on the other hand, the active ingredient concentration in a working solution must not, for environmental reasons, be so high that the active ingredient can then no longer be degraded upon further dilution, e.g. in water-treatment plants.

In addition, after disinfection has been carried out, before the surface disinfected in each case can be used further, it is frequently necessary to remove disinfectant residues; this is particularly important in the case of cleaning or disinfecting in the clinical sector and of containers and/or pipelines in the food-processing industry, and industrial cleaning in continuous flow washing plants. Numerous experiments have, however, shown that quats have an extremely high adhesion to surfaces. In the food industry sector, disinfectants must be rinsed off thoroughly such that only the amounts which are technically unavoidable with reasonable expense remain. In the pharmaceutical industry sector, production in accordance with the GMP guidelines in the course of cleaning validation requires that residues must not remain on treated surfaces following disinfection and cleaning.

Table 1 below gives the typical concentration recommendation for a quat-containing standard commercial concentrate (containing about 5 to 20% by weight of active ingredient), from which a working solution is obtained by dilution with tap water, the concentration depending on the contact time (figures in per cent by weight of concentrate in water as a function of the contact time).

Table 1

|  | 5 min | 30 min | 60 min | 2 h | 4 h |
|---|---|---|---|---|---|
| Surface disinfection in hospitals and doctors' surgeries |  | 1.0% | 0.5% |  | 0.25% |
| HBV/HIV |  | 1.0% | 0.5% | 0.25% |  |
| Rotavirus | 0.25% |  |  |  |  |
| Tuberculosis pathogen |  | 3.0% | 2.5% | 2.0% |  |
| Kitchen and food sector<br>    10°C low-level<br>    contamination<br>    standard contamination |  | 0.25%<br>1.0% | 0.25%<br>0.5% |  |  |
|     20°C low-level<br>    contamination<br>    standard contamination |  | 0.25%<br>0.5% | 0.25%<br>0.25% |  |  |

Although the quats used in disinfectants exhibit only relatively low human toxicity compared with other active ingredients such as aldehydes or active chlorine, side effects cannot be excluded, for which reason residues of quats represent undesired foreign substances in foods and pharmaceuticals.

For these reasons, within the framework of the quality control of disinfection measures, reliable methods are required which permit regular and simple control of the degree of dilution and control of the residues of disinfectants.

However, there are currently no universal analytical methods of determining the degree of dilution irrespective of the type of active ingredient and of the original active ingredient concentration. For example, it has hitherto not been possible to detect cationic surfactants rapidly and reliably. Accordingly, there is a need for a simple method of For example, it has hitherto not been possible to detect cationic surfactants rapidly and reliably. Accordingly, there is a need for a simple method of determining the degree of dilution of solutions which comprise microbicidal active ingredients, for example quaternary ammonium compounds. Such solutions are disinfectant concentrates and working solutions and also rinse (off) solutions.

For the on-site determinations of quat-containing disinfection solutions, so-called quat indicator papers in the form of test strips have hitherto been supplied by the manufacturers. These semiquantitative rapid tests do not, however, permit a sufficiently accurate determination of the degree of dilution of a solution.

WO 98/20094 discloses disinfectant compositions comprising active ingredients and optical brighteners, preferably selected from stilbene and pyralozine derivatives. EP0680694 a method of determining a concentration of microbicides by combining with it a fluorescent additive such as sulfonated naphthalenes or pyrenes.

DE 42 34 466 A1 proposes, for determining the active ingredient concentration using tracers, using a fluorescent dye, measuring the fluorescent dye concentration in the solution by light-optical means and accordingly determining the concentration of the active ingredient from the measured values obtained. However, fluorescence measurements are associated both with high expenditure with regard to apparatus and calibration, and thus high costs.

Accordingly, the object of the present invention is to develop a determination method which permits a rapid and reliable determination of the degree of dilution of working solutions having varying concentrations of active ingredient (combinations). For this purpose, inert tracers are to be incorporated into the formulation, the concentration of which in the corresponding working solutions can be easily determined. The determination and the tracers themselves are subject to the following requirements:

➢ The tracer must be selectively detectable.

➢ The tracer must not reduce the activity of the active ingredient.

➢ The tracer must be chemically inert towards the other ingredients of the disinfectant composition (of the concentrate or of the working solution).

➢ The tracer must be largely dermatologically and toxicologically safe since the cleaning personnel may come into contact during disinfection both with the concentrate and with the working solution.

➢ The tracer must be compatible with all materials with which the concentrate or the working solution come into contact.

➢ The tracer must itself have sufficient long-term stability over the entire service life of the composition (about 3 years), i.e. it must not chemically or photochemically decompose.

➢ The appearance of the composition must not be changed by the tracer, i.e. there must be no changes in colour or odour, in particular of the concentrate, as a result of using the tracer.

➢ The concentration of the microbicidal active ingredient (of the active ingredients) should be determinable in the working solutions with a relative standard deviation of less than 10% over a concentration decade.

➢ The tracer should not significantly increase the formulation costs.

Firstly, it has surprisingly been found that this object is achieved according to the invention by a composition which comprises

a) one or more microbicidal active ingredient(s) and
b) one or more UVA-tracer(s) chosen from benzophenone and its derivatives, anthranilic acid and its derivatives,

and optionally further auxiliaries and/or solvents/solubility promoters.

Secondly, the object is achieved according to the invention by a method of determining the concentration of microbicidal active ingredients, which is characterized in that a UVA-tracer is used together with the active ingredient, the UVA-tracer concentration is determined by absorption spectrophotometry, and the active ingredient concentration is determined therefrom.

Composition according to the invention

The novel type of disinfectant compositions (concentrates, working solutions) differ according to the invention from traditional disinfectant compositions by virtue of the fact that they comprise at least one UVA-tracer.

The microbicidal active ingredients which may be used in the compositions according to the invention are, for example, quaternary ammonium compounds (quats), guanidine derivatives, biguanide derivatives, phenol derivatives, iodine-cleaving compounds, peroxide compounds, alkylamine, glycine derivatives, fatty acids, hydroxycarboxylic acids and organic acid. Of these, quats, guanidine derivatives, biguanide derivatives, glycine derivatives, alkylamines and aldehydes are preferred. Particular preference is given to quats. The object according to the invention is, however, also achieved by compositions which do not comprise quats, see Examples, Preparation No. 5, which comprises a combination of aldehydes.

Compositions according to the invention are preferably in the form of clear solutions.

Particularly preferred standard commercial active ingredients which may be used as microbicidal active ingredients in the compositions according to the invention are given in Table 2 below:

Table 2

| Active ingredient | Alkyl chain length | Trade name | Content [% by wt.] |
|---|---|---|---|
| N-Alkyl-N,N,N-trimethylammonium bromide | 16 | Cetyltrimethylammonium bromide | > 99% active ingredient |
| N-Alkyl-N,N-dimethyl-N-ethylammonium ethylsulphate | 16 | Mecetroniumetilsulphate | 70% active ingredient 30% water |
| N,N-Dialkyl-N,N-dimethylammonium chloride | 10, 10 | Bardac-22 | 50% active ingredient 30% water 20% isopropanol |
| N,N-Dialkyl-N-methyl-N-polyoxyethyleneammonium propionate | 10, 10 | Bardap-26 | 70% active ingredient 28% ethylene glycol 2% water |
| N-Alkyl-N-benzyl-N,N-dimethylammonium chloride | 60-70% $C_{12}$ 30-40% $C_{14}$ | Benzalkonium chloride | > 99% active ingredient |
| N,N-bis(3-Aminopropyl)-N-alkylamine | 12 | Lonzabac-12.100 | > 99% active ingredient |
| N-Alkylpropylenedi-amine-N,N'-bisguanidinium diacetate | 12-14 | Lonzabac GA | 80% active ingredient 10% water 10% isopropanol |

Particularly preferred standard commercial active ingredients which may be used in the compositions according to the invention are alkylbenzyldimethylammonium chloride, dialkyldimethylammonium chloride, alkyldimethylethyl ethosulphate, dialkylmethyloxethylammonium propionate and octenidine dihydrochloride (quaternary compounds), chlorhexidine dihydrochloride and cocopropylenediamineguanidinium diacetate (e.g. Lonzabac® GA, Dodigen® 3558, guanidine derivatives), polyhexamethylene biguanide (e.g. Vantocil® IB, biguanide derivatives), 2-biphenylol (phenol derivative), polyvinylpyrrolidone-iodine (PVP-iodine, iodine-cleaving compound), $H_2O_2$ and peracetic acid (peroxide compound), N,N'-bis(3-aminopropyl)dodecylamino(alkylamine), N- (3-aminopropyl)-N'-$C_{10-16}$-alkylglycine derivatives (glycine derivative), undecylenic acid (fatty acid), tartaric acid, malic acid, citric acid, succinic acid and lactic acid (hydroxycarboxylic acids), glyoxylic acid (organic acid), formaldehyde, succinaldehyde, glyoxal, glutaraldehyde and ethylhexenal (aldehydes). The aldehydes are usually available commercially as aqueous solutions.

Quats can form sparingly soluble precipitates with soaps and other anionic surfactants, and as a result lose microbicidal effectiveness. In a preferred embodiment, the compositions according to the invention which comprise quats are accordingly free from soaps and other anionic surfactants.

Although compositions according to the invention can be prepared which comprise only one microbicidal active ingredient, in particular a quat, compositions according to the invention will, in order to achieve a broadband effect, preferably comprise mixtures of active ingredients. The person skilled in the art will decide on the basis of his expert knowledge which active ingredient combination is particularly advantageous for a desired application.

Preferred combinations of active ingredient groups, optionally with further auxiliary groups and/or solvents/solubility promoter groups are quat/amine, $H_2O_2$/peracetic acid, alkylamine/glycine derivative, quat/aldehyde, quat/aromatic alcohol, quat/amine/glycine derivative, quat/glycine derivative, aldehyde/aromatic alcohol.

The UVA-tracers formulated into the compositions according to the invention have, in the corresponding dilution in the working solution, an absorption maximum or two or more absorption maxima at a wavelength in the range from 320 to 400 nm. Such UVA-tracers may, within the range of the measurement wavelength, have a molar absorption coefficient $\varepsilon$ in the range from 500 to 200,000 $l \cdot mol^{-1} \cdot cm^{-1}$, preferably 3000 to 30,000 $l \cdot mol^{-1} \cdot cm^{-1}$, more preferably 8000 to 15,000 $l \cdot mol^{-1} \cdot cm^{-1}$, e.g. about 10,000 $l \cdot mol^{-1} \cdot cm^{-1}$.

In other words, the absorption of the UVA-tracer in the working solutions should, within the range of the measured wavelength, preferably be at E = 0.01 to 2.0 (according to the Lambert-Beer law: $E = \varepsilon \cdot c \cdot d$).

Such substances are known in the prior art and are also referred to as UV absorbers. UV absorber is the collective term for compounds having marked absorption capacity for ultraviolet radiation, which are used as light protection agents (UV stabilizers) for improving the resistance to light of paints and surface coatings, plastics and rubbers (as anti-ageing agents), glasses (UV filters), packaging materials and other industrial products and also as sunscreens in cosmetic preparations.

The widespread cinnamic acid and salicylic acid derivatives are often exclusively UV-B absorbers and/or strong fluorescent dyes and are preferably not present in the compositions according to the invention.

The UV absorbers used as UVA-tracers according to the invention are generally derivatives of benzophenone, the substituents of which, such as hydroxyl and/or alkoxy groups, are mostly in position 2 and/or 4, and also anthranilic acid. The use thereof as light protection agents in cosmetic products exhibits the toxicological and dermatological acceptability of this class of substance for use as UVA-tracers in the compositions according to the invention.

Table 3 gives an overview of preferred UVA-tracers:

Table 3

| Name | Trivial name | Trade name(s) | $\lambda_{max}$ [nm] |
|---|---|---|---|
| 2-Hydroxy-4-methoxybenzophenone | Benzophenone-3 | Uvinul M 40 Neo Heliopan BB Eusolex 4360 Uvasorb MET/C | 285, 325* |
| Menthyl o-aminobenzoate | Menthyl anthranilate | Neo Heliopan MA | 335 |
| 2-Hydroxy-4-methoxybenzophenone -5-sulphonic acid | Benzophenone-4 | Uvinul MS 40 Uvasorb S5 | 282, 325* |
| 2,2'-Dihydroxy-4,4'-dimethoxybenzophenone | Benzophenone-6 | Uvinul D49 | 282, 340 |
| 2,2'-dihydroxy-4,4'-dimethoxybenzophenone-5,5'-disodium sulphonate | Benzophenone-9 | Uvinul DS49 | 282, 330 |

\* Absorption shoulder

Incorporation of the UVA-tracer to obtain the compositions according to the invention may lead to colour changes. The possible yellow coloration may be caused by the reaction of a keto or an aldehyde group of a UVA-tracer, for example if a benzophenone derivative is used as UVA-tracer, with a primary amine group of a substance in the composition, but also as a result of the reaction of the primary amine group of the UVA-tracer, e.g. if menthyl anthranilate is used as UVA-tracer, with an ingredient which contains keto or aldehyde groups.

However, a yellow coloration can also be caused by the fact that a substance present in the composition according to the invention is itself not stable upon storage, particularly during storage under exposure to light. Due to the active ingredient decomposition, many commercial concentrates comprise small amounts of a blue dye which conceals any yellow coloration which may arise during storage. Due to the presence of such a blue dye, any possible yellow coloration following incorporation of a UVA-tracer in the compositions according to the invention is often not detectable. Secondly, as is shown in the examples, the UVA-tracers in the compositions according to the invention also act as UV stabilizers for the entire composition, as a consequence of which any blue dye is itself protected for longer against UV radiation and can develop its concealing action over a longer period. For this reason, specific precautions to avoid a yellow coloration as a result of incorporating a UVA-tracer as a consequence of its possible reaction with an ingredient will often not be necessary.

On the other hand, however, it is readily possible to formulate compositions according to the invention such that a reaction between ingredient, e.g. active ingredient, and UVA-tracer with yellow coloration is largely excluded from the outset. Accordingly, a first preferred composition is characterized in that it does not comprise a primary amine, and the UVA-tracer is benzophenone or a derivative, in particular 2-hydroxy-4-methoxybenzophenone (benzophenone-3).

A second preferred composition according to the invention is characterized in that it comprises aldehyde and/or ketone, and optionally also quat, and the UVA-tracer is benzophenone or a derivative, in particular benzophenone-3.

A third preferred composition according to the invention is characterized in that it comprises a primary amine, for example the alkylamine, glycine derivative, guanidine derivative and/or biguanide derivative, but preferably neither aldehyde nor ketone, and the UVA-tracer is anthranilic acid or a derivative, in particular menthyl anthranilate.

The UVA-tracer is preferably chosen such that the intrinsic colour of the disinfectant is not changed as a result of the incorporation of the UVA-tracer. Such preferred UVA-tracers do not absorb light in the visible spectral region (400 to 800 nm).

Auxiliaries according to the invention are, for example, surfactants, complexing agents, pH extenders, corrosion inhibitors, dyes and fragrances.

Exemplary surfactants are anionic surfactants, such as sodium stearate, potassium stearate or triethanolamine soaps, sulphonated aromatic hydrocarbons, such as N-alkylbenzenesulphonates (e.g. the $C_{10-13}$-alkylbenzenesulphonic acid sodium salt, Marlon® A 350), sulphonated aliphatic hydrocarbons, such as secondary alkanesulphonates and sulphonated olefins, sulphated fatty alcohols, such as sodium lauryl sulphate, sulphated fatty alcohol polyglycol ethers, such as sodium lauryl polyglycol ether sulphate, sulphonated maleic esters, such as lauryl sulphosucciniate, carboxymethylated fatty alcohol polyglycol ethers, such as lauryl polyglycol ether acetate. Preferred nonionic surfactants are alkyl ethoxylates, alkyl phenol ethoxylates, fatty alcohol polyglycol ethers, fatty acid alkylolamides, fatty acid alkylolamide ethoxylates, fatty amine ethoxylates, alkyl polyglycosides, such as cocoyl polyglycose, lauryl polyglucose, decyl polyglucose or polyalkylene oxide block polymer. Exemplary nonionic surfactants are Marlipal® 013/120 (ethoxylated isotridecyl alcohol having 12 EO units), Plurafac® LF 231 (ethoxylated, butoxylated, methylated aliphatic alcohols), Lutensol® ON 110 and ON 70 (ethoxylated isodecyl alcohol having 11 and 7 EO units respectively) and Rewopal® LA 3 (polyethoxylated lauryl alcohol). Preferred amphoteric surfactants are alkylaminoalkylglycines, betaines or sulphobetaines. It is also possible to use combinations of surfactants which are compatible with one another.

Exemplary complexing agents are ethylenediaminetetraacetic acid and nitrilotriacetic acid, and salts thereof, an exemplary sequestering agent is 2-phosphonobutane-1,2,4-tricarboxylic acid (Bayhibit® AM). To adjust the pH it is possible to use organic acids and/or inorganic acids, such as, for example, hydrochloric acid, phosphoric acid or sulphuric acid. To improve material compatibility, it is possible to use corrosion inhibitors, such as, for example, 1H-benzotriazole, tolyltriazole or mercaptobenzoxazole. Exemplary dyes are Patent Blue V (E131) and Quinoline Yellow (E104).

Solvents/solubility promoters used according to the invention are, for example, water, aliphatic and aromatic alcohols, glycols, glycol mono- and diethers, polyols and polyol mono-, di- and polyethers, and mixtures thereof. Preferred aromatic alcohols are phenoxyethanol, phenethyl alcohol, 2-phenoxy-1-propanol, 1-phenoxy-2-propanol, 3-phenoxy-1-propanol and benzyl alcohol. Exemplary aliphatic alcohols are linear or branched $C_2$- to $C_4$-alcohols, e.g. ethanol, 1-propanol and 2-propanol. Particularly preferred solvents/solubility promoters are water, isopropanol and ethylene glycol, and mixtures thereof.

The statements regarding preferred embodiments made for the preparation according to the invention also apply correspondingly for the concentrate according to the invention and the working solution according to the invention.

The composition according to the invention can be in the form of a concentrate, and then comprises 0.05% by weight to 90% by weight, preferably 0.1% by weight to 80% by weight, more preferably 4% by weight to 70% by weight, of active ingredient(s). Such a concentrate according to the invention can comprise 0.05 to 15% by weight, preferably 0.1 to 8% by weight, more preferably 0.5 to 7% by weight, of UVA-tracers.

Preferred concentrates comprise 5 to 99.5% by weight, preferably 15 to 98% by weight, more preferably 25 to 95% by weight, of water.

Exemplary preferred disinfectants into which UVA-tracers may be incorporated in order to obtain the concentrates according to the invention are the preparations 1 to 5 listed in Table 4 in the examples.

The compositions according to the invention are used for disinfection and/or cleaning purposes. For this, the concentrates according to the invention are diluted accordingly and used as a working solution in the medicinal, in particular in the clinical, sector, and in plant and personnel hygiene in the food, pharmaceutical and cosmetics industry.

Working solutions according to the invention can be obtained, for example, from the concentrates according to the invention by dilution with water. For example, a working solution according to the invention can be obtained by diluting one part by volume of concentrate according to the invention with 5 to 10,000 parts by volume, preferably 50 to 5000 parts by volume, more preferably 100 to 2500 parts by volume, of water. In other words, the working solution according to the invention can comprise 0.02 to 10 g/l, preferably 0.05 to 8 g/l, more preferably 0.1 to 5 g/l, of active ingredient.

The compositions according to the invention, in particular the working solutions, can be used in the disinfection, in particular of surfaces. For this, active ingredient concentrations can be readily determined in solution or on surfaces, in particular it is possible to state the degree of dilution of a disinfection working solution or the success of rinsing measures following use of a disinfectant cleaner. Thus, the determination of disinfectant residues in particular is possible.

Method according to the invention

The present invention further relates to a method of determining the concentration of microbicidal active ingredients, which is characterized in that a UVA-tracer is used together with the active ingredient, and the UVA-tracer concentration is determined by means of absorption spectrophotometry.

The method according to the invention involves the absorption spectrophotometric determination of the concentration of a UVA-tracer in a solution in order to obtain the degree of dilution. A statement about the dilution accuracy is thus possible according to the invention.

The absorption spectrophotometric methods are known in the prior art and appropriate instruments are available commercially. Since a photometer is required in each case for an on-site measurement, in choosing the UVA-tracer, the costs of the photometer which is especially suitable for the chosen wavelength $\lambda_{max}$ of the UVA-tracer are taken into consideration. In this connection, it is possible to use a low-cost single-wavelength photometer, which can be used to measure the absorption at a certain wavelength. Even simple handheld filter photometers permit a quantitative determination with a standard deviation of less than 10% over a concentration decade. In the spectral range from 320 to 400 nm, a halogen lamp is usually used as the light source. In addition, low-cost cuvettes made of silicate glass, polystyrene or polymethylmethacrylate can be used.

In the case of the determination of the active ingredient concentration from the UVA-tracer concentration measured, in the method according to the invention, it is assumed that active ingredient and UVA-tracer have been used in a known ratio. The UVA-tracer concentration can be obtained from the measured absorption of the UVA-tracer-containing solution with the help of a simple calibration. In this connection, it is not necessary to find a linear relationship between UVA-tracer concentration and absorption, since it suffices that, by regression, a mathematical relationship is found for which the standard deviation is below 10%. Preferably, the measured absorption is identical to the concentration (in per cent) of the working solution of concentrate.

In a particularly preferred embodiment of the method according to the invention, the degree of dilution of a composition according to the invention, in particular of a working solution according to the invention, is determined, the composition comprising active ingredient and UVA-tracer.

In another embodiment, a composition, for example a working solution according to the invention, which comprises active ingredient and UVA-tracer, is used for the cleaning or disinfection of containers, such as bottles, small boxes and tanks and/or pipelines. The composition or residues of composition are rinsed off or out using a rinse solution, for example using water, the UVA-tracer concentration remaining in the rinse solution after rinsing off or out is determined by means of absorption spectrophotometry, giving rise to the active ingredient concentration (or the degree of dilution of the concentrate) in the rinse solution. Accordingly, using the method according to the invention, it is possible to optimize the amount of rinse solution required for removing microbicidal active ingredient from the container, the tank and/or the pipeline.

In a further preferred embodiment, the method can be carried out such that a composition which comprises active ingredient UVA-tracer is applied to a surface, and the composition acts for a certain time (it being possible for the solvent of the composition to evaporate) and active ingredient and UVA-tracer remain on the surface. To determine the active ingredient concentration applied to the surface, the applied composition is then rinsed off from the surface using a rinse solution, for example an organic solvent, such as acetone, and the UVA-tracer concentration in the rinse solution is determined by means of absorption spectrophotometry and, therefrom, the active ingredient concentration applied to the surface.

Preferred compositions for use in the method according to the invention are the compositions according to the invention which have already been described (concentrates according to the invention and working solutions according to the invention). The statements regarding preferred embodiments of the composition (concentrate, working solution) made above also apply correspondingly for the compositions used in the method according to the invention.

It is, for example, possible to use a concentrate according to the invention in the method according to the invention. The said use of a concentrate according to the invention can, for example, take place such that the method according to the invention involves the dilution of such a concentrate, as a result of which a working solution is obtained. Working solutions for use in the method according to the invention may be obtained by diluting one part by volume of concentrate with 5 to 10,000 parts by volume, preferably 50 to 5000 parts by volume, more preferably 100 to 2500 parts by volume, of water and, for example, 0.02 to 10 g/l, preferably 0.05 to 8 g/l, more preferably 0.1 to 5 g/l, of active ingredient.

As is illustrated in detail in the examples, dilution of the concentrate (according to the invention) to obtain the working solution (according to the invention), in particular if particularly hard water is used for the dilution (e.g. 40° German hardness) may result in the appearance of clouding. The appearance of clouding in working solutions and the abovementioned rinse solutions is independent of the presence of UVA-tracers, but prevents or restricts the implementation of the invention since absorption spectrophotometry is only possible without problems if solutions are clear. The clouding can be removed by adding a nonionic surfactant, for example an alkylphenol ethoxylate, such as, for example, Marlophen® 850 (p-isononylphenol ethoxylate with a degree of ethoxylation of 50).

Since the absorption of solutions which comprise UVA-tracers may be dependent on the pH, it is preferred, prior to the absorption spectrophotometric measurement, to establish, by adding acid, base or buffer, a desired pH of the solution, of which the UVA-tracer concentration is to be determined by means of absorption spectrophotometry.

The invention is based on the fact that it has surprisingly been found that microbicidal active ingredients in the dilutions in which they are used for disinfection exhibit such a low absorption in the wavelength range from 320 to 400 nm that it is possible, using UVA-tracer substances, to determine indirectly the active ingredient concentration in solutions which contain active ingredients. In this respect, the invention affords the following advantages:

> ➤ The UVA-tracers can be incorporated by the disinfectant manufacturers, for example the manufacturers of concentrates and using low-cost apparatus.

> ➤ The required amount of UVA-tracer is small and does not impair the microbicidal activity of the active ingredient.

> ➤ The required amount of UVA-tracer brings about low material costs.

> ➤ The UVA-tracers are, as standard commercial substances (UV absorbers), readily available and available in a wide selection at comparatively low cost.

> ➤ It is possible to use UVA-tracers which have a storage-stabilizing action, in particular during storage under the effect of light.

> ➤ The measurement method on which the invention is based (absorption spectrophotometry) is low cost in terms of apparatus and there are commercial low cost measurement instruments which have low susceptibility to failure.

> ➤ The instruction of personnel on site (of service technicians in the course of metering instrument checks and also of hospital personnel in the course of hygiene monitoring) is possible with a low time expenditure.

> ➤ Determinations of active ingredient concentrations are possible with a relative standard deviation of less than 10%.

> ➤ The UV-A-tracers are for the most part dermatologically and toxicologically safe.

> ➤ It is easy to find UV-A-tracers which are chemically inert towards the ingredients of the disinfectants.

> ➤ It is possible to find UV-A-tracers which do not change the optical or odiferous properties of the disinfectants.

The invention is illustrated in more detail by the examples below.

**Examples**

Percentages are percentages by weight.

The water-based concentrates referred to in the examples below by preparation 1 to 5 are:

Table 4

| Prep. No. | Active ingredients/active ingredient combinations | Selected ingredients[1] | Proportion (% by wt.)[2] |
|---|---|---|---|
| 1 | Quat + alkylamine | Benzalkonium chloride | 10 |
| | | N,N-bis(3-aminopropyl)-N-dodecylamine | 3 |
| | | Phenoxypropanols | 1.5 |
| | | Nonionic surfactants | 8 |
| 2 | Quat + GuAc | Benzalkonium chloride | 25 |
| | | Cocopropylenediaminebis-guanidinium diacetate | 1.4 |
| | | Phenoxyethanol | 1.8 |
| | | Isopropanol | 5 |
| | | Nonionic surfactants | 20 |
| 3 | Quat + org. acid | Benzalkonium chloride | 15 |
| | | Glyoxylic acid | 15 |
| | | Phenoxypropanols | 5 |
| | | Nonionic surfactants | 5 |
| 4 | Combinations of aldehydes | Formaldehyde | 9 |
| | | Glyoxal | 14 |
| | | Glutaraldehyde | 2 |
| | | Ethanol | 7.7 |
| | | Linear alkylbenzene-sulfonate (LAS) | 7.5 |
| | | Nonionic surfactants | 3 |
| | | Blue dye | 0.005 |
| | | Yellow dye | 0.005 |
| 5 | Quat + phenoxypropanols | Benzalkonium chloride | 20 |
| | | Phenoxypropanols | 35 |
| | | Nonionic surfactants | 12.5 |
| | | Isopropanol | 5 |
| | | N,N,N',N'-tetrakis-(hydroxypropyl) ethylenediamine | 4 |
| | | Yellow dye | 0.001 |
| | | Blue dye | 0.001 |

[1] Customary auxiliaries and solvents/solubility promoters are also present; [2] % by wt, of active constituent.

In the examples, the names of the UV-A-tracers used follow Table 3.

**Example 1**

**Solubility of selected UV-A-tracers in selected concentrates**

To check the solubility of selected UV-A-tracers in traditional concentrates containing various active ingredient combinations, 100 g of preparations 1 to 5 (concentrate) were in each case treated with 0.2 g of UV-A-tracer and stirred intensively over a period of 3 hours. The results of these formulation experiments are shown in Table 5:

Table 5

|  | Prep.1 | Prep.2 | Prep.3 | Prep.4 | Prep.5 |
|---|---|---|---|---|---|
| Benzophenone-3 | +y | +y | + | + | + |
| Menthyl anthranilate | + | + | + | + | + |
| Benzophenone-4 | +y | +y | + | + | + |
| Benzophenone-6 | +y | +y | +y | + | + |
| Benzophenone-9 | +y | +y | +y | + | + |

Solubilities of selected UV-A-tracers in concentrates containing various active ingredient combinations (preparations 1 to 5) and optically visible changes compared with the original concentrate, "+" UV-A-tracer soluble in 100 g of preparation to at least 0.2 g, "y" the originally colourless concentrate turns yellowish as a result of the incorporation of the UV-A-tracer.

Benzophenone-3 dissolves without problems in all five concentrates, although in preparations 1 and 2 an intensive yellow coloration of the originally colourless preparations can be seen. Spectroscopic control measurements on these preparations have displayed a significant shifting of the absorption shoulder of benzophenone-3 in preparations 1 and 2. Since this absorption shifting is only observed in the amine-containing preparations 1 and 2, it is to be assumed that the primary amines react with the keto group of benzophenone-3 to form azomethines (Schiff's bases). Benzophenone-3 is therefore preferably only used as a tracer in disinfectants which do not contain primary amines.

Menthyl anthranilate dissolves in all preparations without visible colour changes.

Benzophenone-4, benzophenone-6 and benzophenone-9 likewise display an intensive yellow coloration in amine-containing preparations. However, compared with benzophenone-3, said three benzophenone derivatives are relatively expensive and have a significantly lower absorption coefficient in the absorption maximum, for which reason they have not been considered further as UV-A-tracers below for reasons of cost.

**Example 2**

**Stability investigations of UV-A-tracer-containing concentrates**

Since the UV-A-tracers are to be used for determining the active ingredient concentrations of disinfectant solutions, the UV-A-tracer-containing concentrates must be sufficiently stable throughout the entire storage life of the commercially available disinfectant concentrates (approximately 3 years). In order to investigate the stability of the UV-A-tracers, preparations 1 to 5 to which UV-A-tracers have been added were stored in clear glass bottles at room temperature firstly on a window-sill and secondly as a reference in the dark. Over a period of 3 months, the optically visible changes of the UV-A-tracer-containing preparations stored under varying conditions were observed and, at regular time intervals, 1% dilutions of these UV-A-tracer-containing preparations in water were investigated spectroscopically. The results are given in Table 6.

Table 6

| UV-A-tracer | Disinfectant | Conc. [g/l]* | Changes within the observation period |
|---|---|---|---|
| Benzophenone-3 | Preparation 3 | 1.00 | none |
|  | Preparation 4 | 1.14 | green → blue-green |
|  | Preparation 5 | 1.02 | green → yellow-green |
| Menthyl anthranilate | Preparation 1 | 2.83 | slight yellow coloration |
|  | Preparation 2 | 2.00 | slight yellow coloration |
|  | Preparation 3 | 3.65 | intensive yellow coloration |
|  | Preparation 4 | 3.35 | green → yellow-green |
|  | Preparation 5 | 3.58 | green → yellow |

*Concentration of the UV-A-tracer

a) **Benzophenone-3**

Under the effect of light, in the case of preparation 5 there is a slight colour change from green to blue-green and in the case of preparation 4 from green to yellow-green. By contrast, preparation 3 does not display changes of any kind. The slight colour changes within the green shade can be observed both in the case of preparation 4 and also in the case of preparation 5 only after about 6 to 8 weeks and are so slight that they can only be recognized in a direct comparison. Since no discolorations were found in the case of colourless preparation 3, it was suspected that the dyes in the original preparations 1 to 5 (without the addition of UV-A-tracer) were able to decompose under the effects of light and are thus responsible for the colour changes.

In order to check this, the preparations 1 to 5 (without UV-A-tracers) were, for comparison, likewise exposed to daylight on a window-sill. Here, it was found that the green preparation 5 became yellow within 8 hours, by which a photochemical decomposition of the dye B (absorption maximum at $\lambda_{max}$ = 639 nm) present therein can be assumed. Preparation 4 contains the same dyes as preparation 5, although the concentration of these dyes is significantly higher in preparation 4, meaning that the yellowing only arises later (after approximately 6 weeks). In the case of the other disinfectants, no colour changes were observed.

Decomposition of the blue dye under the effect of light is an explanation for the slight yellowing of preparation 4, but not for the colour change in the case of preparation 5. While the pure preparation 5 becomes yellow within 24 hours under the effect of light, in the case of the preparation 5 treated with benzophenone, a slight coloration from green to blue-green arises, although this is only visible after a few weeks. The UV-A-tracer benzophenone-3 thus acts as UV-absorber and protects the preparations 4 and 5 against the rapid photochemical decomposition of the blue dye.

b) **Menthyl anthranilate**

Under the effect of light, a slight yellowish tinge can be established in the case of preparations 1 and 2 after 3 months; however, this is so slight that it can only be recognized if the samples stored under varying conditions are compared directly. By contrast, the originally green preparations 4 and 5 change colour to a significant extent. Preparation 5 changes from green to yellow and preparation 4 adopts a pale yellow-green colour. It is to be assumed that these colour changes can likewise be attributed to the inadequate photostability of the dye in the preparations 4 and 5, and not to the UV-A-tracer itself. The UV-A-tracer menthyl anthranilate acts, like benzophenone-3 as well, as UV-absorber and delays the photochemical decomposition of the blue dye to a significant extent, although it is unable to prevent it completely.

The observed coloration of the green preparations 4 and 5 upon exposure to daylight is to be attributed not to the UV-A-tracers, but to the inadequate photostability of the dyes originally present in the preparation. On the contrary, the addition of the tracers can even result in a significant improvement in the photostability of these disinfectants being achieved.

**Example 3**

**Absorption spectrophotometric stability investigations of the incorporated UV-A-tracers in concentrate and working solution**

The long-term stability of combinations of concentrate and UV-A-tracers in the dark and under the effect of light was investigated by reference to UV spectra. The combinations below were used (Table 7):

Table 7

|  | UV-A-tracer | Content [g/l] |
|---|---|---|
| Preparation 1 | Menthyl anthranilate | 2.83 |
| Preparation 2 | Menthyl anthranilate | 2.00 |
| Preparation 3 | Benzophenone-3 | 1.00 |
| Preparation 4 | Benzophenone-3 | 1.14 |
| Preparation 5 | Benzophenone-3 | 1.02 |
|  | Menthyl anthranilate | 3.58 |

Following storage at room temperature for 4, 8 or 12 weeks in the dark or in daylight, 1% strength dilutions of each of the preparations in water were prepared.

**Result:**

**a) Benzophenone-3 (preparations 3, 4 and 5)**

In the case of all three combinations of preparation and UV-A-tracer, the absorptions (of the 1% strength dilutions) measured move within a tolerance limit of $\pm$ 10%, based on the initial absorption. The fluctuations with respect to time throughout the observed 12 weeks are in the case of preparation 3 about $\pm$ 2%, in the case of 4 according to the invention about $\pm$ 3% and in the case of preparation 5 about $\pm$ 9%.

The comparison between the samples which have been stored in the dark and in daylight revealed differences of at most $\pm$ 2% in all cases for the same storage period. Thus, the fluctuations with respect to time are greater than a photochemical effect as a result of exposure to daylight. In none of the three disinfectants was decomposition of benzophenone-3 observed; this is true both for storage in the dark and also for storage in daylight.

**b) Menthyl anthranilate (Preparations 1, 2 and 5)**

The fluctuations of the absorptions (of the 1% strength dilutions in water) with respect to time are in the case of preparations 1 and 5 about $\pm$ 5% and in the case of preparation 2 about $\pm$ 3%.

The stability investigations of the combination of preparation 5 with menthyl anthranilate showed, after 12 weeks, a significant divergence of the absorptions (of the 1% strength dilutions in water) between the menthyl anthranilate-containing sample stored in the dark and that exposed to daylight. At the same time, an intensive unpleasant odour is observed in the case of this latter sample. A comparison sample without UV-A-tracer likewise exposed to daylight had this odour after just 10 weeks, meaning that the UV-A-tracer acts as UV-absorber and even also counteracts the photochemical decomposition of preparation 5. By contrast, the sample stored in the dark does not display any perceptible changes. Exposure to daylight over several months is a particularly drastic method and (in particular in the case of good stability during the more realistic storage in the dark) should not be overrated. The menthyl anthranilate in preparation 5 is, in addition to benzophenone-3, a possible UV-A-tracer.

Accordingly, benzophenone-3 can be used as a UV-A-tracer in quat- and aldehyde-containing disinfectants which do not contain amines (e.g. N,N-bis(3-aminopropyl)dodecylamine or coco-guanidinium diacetate), while menthyl anthranilate can be used in amine-containing preparations. Glyoxylic acid reacts with menthyl anthranilate, meaning that in preparation 3, benzophenone-3 should be used exclusively. Only in preparation 5 is the sensible use of both UV-A-tracers possible.

Since disinfectant active ingredient combinations of amines with aldehydes or acids are often not possible in any case for reasons of compatiblity, by reference to the investigations presented here it is possible, depending on the disinfectant composition (quat-containing, aldehyde-containing, amine-containing and/or acidic disinfectants) to choose a particularly suitable UV-A-tracer for the photometric concentration determination according to the invention of solutions containing active ingredients.

**Example 4**

**Investigations of troublesome effects relevant in practice (pH, water hardness)**

Since tap water can have very different water hardnesses depending on the region, the effect of water hardness on the photometric determination was investigated. For this, all preparations 1 to 5 treated with UV-A were diluted, instead of with distilled water, with tap water of 15 to 20° German hardness, and additionally $CaCl_2$ was added in an amount such that the calculated water hardness was approximately 40° German hardness. In this respect, it was found that a number of disinfectant dilutions exhibited greater or lesser cloudings and were thus not accessible for a direct photometric determination.

In order to rule out the clouding not being caused by a possible precipitating-out of the incorporated UV-A-tracers, the corresponding dilutions of the UV-A-tracer-free preparations were prepared. It was found that the cloudings are not attributable to the incorporated UV-A-tracers.

The effect of temperature on the photometric determination of the preparation dilutions was investigated in the temperature range between 5°C and 30°C since the temperature at which the analyses are carried out can vary between these values depending on the analysis site and time of year. For this, various disinfectant dilutions of the concentrates preparation 3 (plus benzophenone-3) and preparation 5 (plus menthyl anthranilate) were measured, for example, at 5°C, 18°C and 30°C. In the investigated temperature range, the measurements have in all cases produced deviations in the absorption ($\Delta E$) of significantly less than 1%. The effect of temperature on the measurements can therefore be disregarded.

In order to investigate the effect of pH on the photometric determination, 1% dilutions of the disinfectant preparations 1 (plus menthyl anthranilate), preparation 4 (plus benzophenone-3) and preparation 5 (plus benzophenone-3) in distilled water were prepared in each case. Then, by adding sodium hydroxide solution or hydrochloric acid, pH values between pH = 1 to pH = 13 were established.

While in the case of the UV-A-tracer benzophenone-3, the absorption decreases significantly with increasing pH both in the case of preparation 4 and also in the case of preparation 5, in the case of the preparation 1 treated with menthyl anthranilate, a significant absorption increase can be observed. Accordingly, with regard to a simple and uniform photometric determination in solutions by means of UV-A-tracers, the pH of the solutions should be 7 or less.

To dissolve precipitates which may arise in cases of dilution with hard water, extensive solubility tests were carried out, which showed that nonionic surfactants, in particular alkylphenol ethoxylates (compared with alkyl ethoxylates, sorbitan esters and alkyl polyglycosides) have the best dissolution properties for these precipitates. The nonionic surfactant which proved to have the best dissolution properties was Marlophen® 850 from Hüls AG, Marl (p-inonylphenyl ethoxylate with a degree of ethoxylation EO of 50), which dissolves in water up to 10% to form a clear solution.

Marlophen® 850 satisfies all requirements placed on a measurement auxiliary. The surfactant is able to dissolve the precipitates which arise as a result of the use of hard water within a few seconds, and an addition of just 5 mg in a cuvette filled with 7 ml of preparation dilution suffices for this purpose. By virtue of the nonionic character of the surfactant, Marlophen® 850 is also compatible with the ingredients of all disinfectants. It shows no absorption above $\lambda$ = 300 nm, as a result of which, from a UV-A-absorption spectrophotometric viewpoint, no disturbances are to be expected. Furthermore, it is toxicologically safe, meaning that no particular safety measures are required for its handling.

Citric acid was chosen to adjust the pH. The water-soluble organic acid is inexpensive and safe from a toxilocological viewpoint. The amount of citric acid to be used was determined such that, following the addition of the acid (irrespective of the preparation used and the compound used), the pH is $\leq$ 6.5.

In the real sample measurements of working solutions shown in Example 5, an aqueous solution of the following composition was used:

2 g of Marlophen® 850 + 0.5 g of citric acid + 20 g of

dist. Water.

In each case 50 $\mu$l of this solution were required in order - irrespective of the preparation used and the dilution used - to establish, in a cuvette filled with 7 ml, a pH of less than 6.5, and to dissolve the cloudings. Following the addition, the contents of the cuvette were briefly mixed by shaking.

**Example 5**

**Real sample measurements of working solutions**

0.1 to 1.4% strength dilutions of all preparations 1 to 5 treated with the UV-A-tracers were prepared with hard tap water (approximately 40° German hardness). The photometric analyses were carried out both using a spectrophotometer (CADAS 100, manufacturer: Dr Lange) uniformly at $\lambda$ = 340 nm, and also using a hand filter photometer (LASA plus, Dr Lange) which contains an optical coloured glass grating with $\lambda_{max} \approx$ 330 nm. All measurements were carried out in round disposable glass cuvettes (d approximately 11.0 mm) against tap water as the blank value.

50 $\mu$l of the acidic Marlophen® 850 solution were added to each of the dilutions using a pipette, and the solution was measured.

For the evaluation, the three following regressions were carried out in each case:

1) Linear regression of the type $y = B * x$ (i.e. regression through the origin of coordinates (0;0) through all measurement points measured using a spectrophotometer following the addition of the acidic Marlophen solution.

2) Polynomial regression of the type $y = A + B * x + B_2 * x^2$ (i.e. 2nd order) through all measurement points measured using a filter photometer following the addition of the acidic Marlophen solution.

3) Linear regression of the type $y = A + B * x$ through all measurement points in the range between 0.2% and 1.0% measured using a filter photometer following the addition of the acidic Marlophen solution.

In each case the UV-A-tracers given were incorporated into the preparations, and then the dilutions to 0.1, 0.2, 0.3, 0.4, 0.6, 0.8, 1.0, 1.2 and 1.4% by weight of UV-A-tracer-containing preparation in water were prepared.

The regression data are summarized in Table 8.

Table 8

| | | Prep. 1** | Prep. 2** | Prep. 3* | Prep. 4* | Prep. 5* | Prep. 5** |
|---|---|---|---|---|---|---|---|
| UV-A-tracer in the preparation [g/kg] | | 5.38 | 5.46 | 3.00 | 2.91 | 3.82 | 5.40 |
| CADAS 100[1] (n=9) | B | 1.074 | 1.069 | 1.111 | 1.108 | 1.408 | 1.063 |
| | R | 0.9993 | 0.9994 | 0.9999 | 0.9999 | 0.9999 | 0.9995 |
| | sd | 0.023 | 0.020 | 0.008 | 0.007 | 0.007 | 0.017 |
| LASA plus[2] (n=9) | A | 0.005 | -0.014 | 0.018 | 0.013 | 0.000 | -0.018 |
| | $B_1$ | 0.848 | 0.886 | 0.674 | 0.687 | 0.881 | 0.890 |
| | $B_2$ | -0.101 | -0.111 | -0.108 | -0.110 | -0.185 | -0.134 |
| | R | 0.9995 | 0.9999 | 0.9998 | 0.9990 | 0.9998 | 0.9999 |
| | sd | 0.003 | 0.002 | 0.004 | 0.009 | 0.004 | 0.002 |
| LASA plus[3] (n=6) | A | 0.034 | 0.016 | 0.043 | 0.047 | 0.056 | 0.021 |
| | B | 0.726 | 0.753 | 0.553 | 0.551 | 0.652 | 0.727 |
| | R | 0.9995 | 0.9994 | 0.9986 | 0.9974 | 0.9976 | 0.9992 |
| | sd | 0.008 | 0.009 | 0.010 | 0.014 | 0.016 | 0.010 |

*Tracer: Benzophenone-3

**Tracer: Menthyl anthranilate

[1] Linear regression through the origin of coordinates: $y = B*x$

[2] Polynomial regression 2nd order: $y = A + B_1*x + B_2*x^2$

[3] Linear regression between 0.2% and 1.0%: $y = A + B*x$.

All of the measurements using the spectrophotometer produce, following the addition of the acidic Marlophen® 850 solution, calibration curves with good correlation coefficients (R > 0.999) and standard deviations (sd < 0.025). While the preparations treated with benzophenone-3 have, upon dilution, excellent standard deviations of sd ≤ 0.01, the calibrations in the case of menthyl anthranilate exhibit a slight curvature over the entire concentration range, meaning that the standard deviations are between sd = 0.007 and 0.023. A concentration determination using a spectrophotometer at λ = 340 nm is possible with adequate accuracy (< 10%) for benzophenone-3 and menthyl anthranilate following the addition of the acidic surfactant solution. Since the calibration curves investigated pass through the origin of coordinates, the measured absorptions are directly proportional to the standardized dilutions.

The calibrations using the filter photometer are curved because of the half-width of the optical glass colour filter of about 70 nm. A linear calibration over the measured range from 0.1% to 1.4% is therefore not possible with the desired accuracy. In all cases, a polynomial regression of the second order produced good correlation coefficients of R ≥ 0.999 and an excellent standard deviation of sd < 0.01. The linear regression in the range between 0.2% and 1.0%, for which the measurement values at 0.1%, 1.2% and 1.4% were not taken into consideration, led to calibration curves with good correlation coefficients (R > 0.997) and good standard deviations (sd ≤ 0.02), meaning that, for the required concentration range, a simple hand filter photometer with a linear evaluation unit can be used for the concentration determination.

**Claims**

1. Composition **characterized in that** it comprises

   a) one or more microbicidal active ingredient(s) and
   b) one or more UVA-tracer(s) chosen from benzophenone and its derivatives, anthranilic acid and its derivatives,

and optionally further auxiliaries and/or solvents/solubility promoters.

2. Composition according to Claim 1, **characterized in that** the active ingredient is chosen from quaternary ammonium compound, guanidine derivative, biguanide derivative, glycine derivative, aldehyde and alkylamine.

3. Composition according to Claim 2, **characterized in that** the quaternary ammonium compound is chosen from alkylbenzyldimethylammonium chloride, dialkyldimethylammonium chloride, alkyldimethylethyl ethosulphate, dialkylmethyloxethylammonium propionate and octenidine dihydrochloride.

4. Composition according to Claim 2, **characterized in that** the guanidine derivative is chosen from chlorhexidine dihydrochloride and cocopropylenediamineguanidinium diacetate.

5. Composition according to Claim 2, **characterized in that** the glycine derivative is an N-(3-aminopropyl)-N' -C$_{10-16}$-alkylglycine derivative.

6. Composition according to Claim 2, **characterized in that** the biguanide derivative is polyhexamethylenebiguanide.

7. Composition according to Claim 2, **characterized in that** the aldehyde is chosen from formaldehyde, succinaldehyde, glyoxal, glutaraldehyde and ethylhexanal.

8. Composition according to Claim 2, **characterized in that** the alkylamine is N,N'-bis(3-aminopropyl) dodecylamine.

9. Composition according to one of Claims 1 to 8, **characterized in that** the UVA-tracer is chosen from 2-hydroxy-4-methoxybenzophenone, menthyl anthranilate, methyl anthranilate, ethyl anthranilate and derivatives of the above, in particular menthyl anthranilate and 2-hydroxy-4-methoxybenzophenone.

10. Composition according to one of Claims 1 to 9, **characterized in that** the auxiliary is chosen from surfactants, complexing agents, pH extenders, corrosion inhibitors, dyes and fragrances, and mixtures thereof.

11. Composition according to one of Claims 1 to 10, **characterized in that** the solvent/the solubility promoter is chosen from water, aliphatic and aromatic alcohols, glycols, glycol mono- and diethers, polyols and polyol mono-, di- and polyethers, in particular water, isopropanol and ethylene glycol, and mixtures thereof.

12. Composition according to one of Claims 1 to 11, **characterized in that**, within the range of the measured wavelength, the molar absorption coefficient $\varepsilon$ of the UVA-tracer is in the range from 500 to 200,000 l $\cdot$ mol$^{-1}$ $\cdot$ cm$^{-1}$, preferably 3000 to 30,000 l $\cdot$ mol$^{-2}$ $\cdot$ cm$^{-1}$, more preferably 8000 to 15,000 l $\cdot$ mol$^{-1}$ $\cdot$ cm$^{-1}$, in particular about 10,000 l $\cdot$ mol$^{-1}$ $\cdot$ cm$^{-1}$.

13. Composition according to one of Claims 1 to 13, **characterized in that** it comprises aldehyde and/or ketone, and the UVA-tracer is benzophenone or a derivative, in particular 2-hydroxy-4-methoxybenzophenone.

14. Composition according to one of Claims 1 to 13, **characterized in that** the weight ratio (wt./wt.) of active ingredient to UVA-tracer is in the range from 2000:1 to 1:300, preferably 850:1 to 1:80 and more preferably 170:1 to 1:50.

15. Composition according to one of Claims 1 to 14 in the form of a concentrate, **characterized in that** the concentrate comprises 0.05% by weight to 90% by weight, preferably 0.1% by weight to 80% by weight, more preferably 4% by weight to 70% by weight, of active ingredient.

16. Concentrate according to Claim 15, **characterized in that** it comprises 0.05 to 15% by weight, preferably 0.1 to 8% by weight, more preferably 0.5 to 7% by weight, of UVA-tracer.

17. Use of a composition according to one of Claims 1 to 16 for disinfection and/or cleaning, in particular surface disinfection, surface cleaning and instrument disinfection.

18. Use of a composition according to one of Claims 1 to 16 to determine active ingredient concentrations in solution or on surfaces, in particular to determine the degree of dilution.

19. Method of determining the concentration of microbicidal active ingredients in a composition which is a working solution obtained by diluting one part by volume of concentrate according to Claim 15 or 16 with 5 to 10,000 parts by volume, preferably 50 to 5000 parts by volume, more preferably 100 to 2500 parts by volume, of water, **characterized in that** the UVA-tracer concentration is determined by means of absorption spectrophotometry.

20. Method according to Claim 19, **characterized in that** the composition which comprises active ingredient and UVA-tracer is rinsed off or out using a rinse solution and the UVA-tracer concentration remaining in the rinse solution after rinsing off or out is determined by means of absorption spectrophotometry.

21. Method according to Claim 20, **characterized in that** a composition which comprises active ingredient and UVA-tracer is applied to a surface, the applied composition is rinsed off from the surface using a rinse solution, and the UVA-tracer concentration in the rinse solution is determined by means of absorption spectrophotometry.

22. Method according to Claim 19, **characterized in that** the working solution comprises 0.02 to 10 g/l, more preferably 0.05 to 8 g/l, more preferably 0.1 to 5 g/l, of active ingredient.

23. Method according to one of Claims 19 to 22, **characterized in that** the solution whose UVA-tracer concentration is to be determined by means of absorption spectrophotometry is treated, prior to the absorption spectrophotometric measurement, with a nonionic surfactant, in particular an alkylphenol ethoxylate.

24. Method according to one of Claims 19 to 23, **characterized in that** the solution whose UVA-tracer concentration is to be determined by means of absorption spectrophotometry, is converted, prior to the absorption spectrophotometric measurement, to a desired pH by adding acid, base or buffer.

**Patentansprüche**

1. Zusammensetzung, **dadurch gekennzeichnet, dass** sie

    a) einen oder mehrere mikrobizide Wirkstoff(e) und

    b) einen oder mehrere UV-A-Tracer, ausgewählt aus Benzophenon und seinen Derivaten, Anthranilsäure und ihren Derivaten,

    sowie gegebenenfalls weitere Hilfsstoffe und/oder Lösungsmittel/Lösungsvermittler umfasst.

2. Zusammensetzung nach Anspruch 1, **dadurch gekennzeichnet, dass** der Wirkstoff ausgewählt ist aus quartäre Ammoniumverbindung, Guanidin-Derivat, Biguanid-Derivat, Glycin-Derivat, Aldehyd und Alkylamin.

3. Zusammensetzung nach Anspruch 2, **dadurch gekennzeichnet, dass** die quartäre Ammoniumverbindung ausgewählt ist aus Alkylbenzyldimethylammoniumchlorid, Dialkyldimethylammoniumchlorid, Alkyldimethylethylethosulfat, Dialkylmethyloxethylammoniumpropionat und Octenidindihydrochlorid.

4. Zusammensetzung nach Anspruch 2, **dadurch gekennzeichnet, dass** das Guanidin-Derivat ausgewählt ist aus Chlorhexidindihydrochlorid und Cocospropylendiaminguanidiniumdiacetat.

5. Zusammensetzung nach Anspruch 2, **dadurch gekennzeichnet, dass** das Glycin-Derivat ein N-(3-Aminopropyl)-N'-C$_{10-16}$-alkylglycin-Derivat ist.

6. Zusammensetzung nach Anspruch 2, **dadurch gekennzeichnet, dass** das Biguanid-Derivat Polyhexamethylenbiguanid ist.

7. Zusammensetzung nach Anspruch 2, **dadurch gekennzeichnet, dass** der Aldehyd ausgewählt ist aus Formaldehyd, Succindialdehyd, Glyoxal, Glutardialdehyd und Ethylhexanal.

8. Zusammensetzung nach Anspruch 2, **dadurch gekennzeichnet, dass** das Alkylamin N,N'-Bis-(3-aminopropyl)-dodecylamin ist.

9. Zusammensetzung nach einem der Ansprüche 1 bis 8, **dadurch gekennzeichnet, dass** der UV-A-Tracer ausgewählt ist aus 2-Hydroxy-4-methoxybenzophenon, Anthranilsäurementhylester, Anthranilsäuremethylester, Anthranilsäureethylester und Derivaten der genannten, insbesondere Anthranilsäurementhylester und 2-Hydroxy-4-methoxybenzophenon.

**10.** Zusammensetzung nach einem der Ansprüche 1 bis 9, **dadurch gekennzeichnet, dass** der Hilfsstoff ausgewählt ist aus Tensiden, Komplexbildnern, pH-Stellmitteln, Korrosionsinhibitoren und Farb- und Duftstoffen, sowie deren Mischungen.

**11.** Zusammensetzung nach einem der Ansprüche 1 bis 10, **dadurch gekennzeichnet, dass** das Lösungsmittel/der Lösungsvermittler ausgewählt ist aus Wasser, aliphatischen und aromatischen Alkoholen, Glykolen, Glykolmono- und -diethern, Polyolen und Polyolmono-, -di- und -polyethern, insbesondere Wasser, Isopropanol und Ethylenglykol, sowie deren Mischungen.

**12.** Zusammensetzung nach einem der Ansprüche 1 bis 11, **dadurch gekennzeichnet, dass** im Bereich der Messwellenlänge der molare Extinktionskoeffizient $\varepsilon$ des UV-A-Tracers im Bereich von 500 bis 200 000 l

- $mol^{-1} \cdot cm^{-1}$, bevorzugt 3000 bis 30 000 l
- $mol^{-1} \cdot cm^{-1}$, bevorzugter 8000 bis 15 000 l
- $mol^{-1} \cdot cm^{-1}$, insbesondere etwa 10 000 l
- $mol^{-1} \cdot cm^{-1}$, liegt.

**13.** Zusammensetzung nach einem der Ansprüche 1 bis 12, **dadurch gekennzeichnet, dass** sie Aldehyd und/oder Keton umfasst und der UV-A-Tracer Benzophenon oder ein Derivat, insbesondere 2-Hydroxy-4-methoxybenzophenon, ist.

**14.** Zusammensetzung nach einem der Ansprüche 1 bis 13, **dadurch gekennzeichnet, dass** das Gewichtsverhältnis (Gew./Gew.) von Wirkstoff zu UV-A-Tracer im Bereich von 2 000 : 1 bis 1 : 300, bevorzugt 850 : 1 bis 1 : 80 und bevorzugter 170 : 1 bis 1 : 50, liegt.

**15.** Zusammensetzung nach einem der Ansprüche 1 bis 14 in Form eines Konzentrats, **dadurch gekennzeichnet, dass** das Konzentrat 0,05 Gew.-% bis 90 Gew.-%, bevorzugt 0,1 Gew.-% bis 80 Gew.-%, bevorzugter 4 Gew.-% bis 70 Gew.-% Wirkstoff umfasst.

**16.** Konzentrat nach Anspruch 15, **dadurch gekennzeichnet, dass** es 0,05 bis 15 Gew.-%, bevorzugt 0,1 bis 8 Gew.-%, bevorzugter 0,5 bis 7 Gew.-%, UV-A-Tracer umfasst.

**17.** Verwendung einer Zusammensetzung nach einem der Ansprüche 1 bis 16 zur Desinfektion und/oder Reinigung, insbesondere der Flächendesinfektion, Flächenreinigung und Instrumentendesinfektion.

**18.** Verwendung einer Zusammensetzung nach einem der Ansprüche 1 bis 16, um Wirkstoffkonzentrationen in Lösung oder auf Flächen zu bestimmen, insbesondere um den Verdünnungsgrad zu bestimmen.

**19.** Verfahren zur Bestimmung der Konzentration von mikrobiziden Wirkstoffen in einer Zusammensetzung, die als Gebrauchslösung vorliegt, die durch Verdünnen eines Volumenteils Konzentrat nach Anspruch 15 oder 16 mit 5 bis 10 000 Volumenteilen, bevorzugt 50 bis 5000 Volumenteilen, bevorzugter 100 bis 2500 Volumenteilen, Wasser erhalten wird, **dadurch gekennzeichnet, dass** die UV-A-Tracer-Konzentration absorptionsspektralphotometrisch bestimmt wird.

**20.** Verfahren gemäß Anspruch 19, **dadurch gekennzeichnet, dass** die Zusammensetzung, die Wirkstoff und UV-A-Tracer umfasst, mit einer Spüllösung ab- bzw. ausgespült wird und die nach Ab- bzw. Ausspülen in der Spüllösung verbliebene UV-A-Tracer-Konzentration absorptionsspektralphotometrisch bestimmt wird.

**21.** Verfahren nach Anspruch 20, **dadurch gekennzeichnet, dass** auf eine Fläche eine Zusammensetzung, die Wirkstoff und UV-A-Tracer umfasst, aufgetragen wird, die aufgetragene Zusammensetzung von der Fläche mit einer Spüllösung abgespült und die UV-A-Tracer-Konzentration in der Spüllösung absorptionsspektralphotometrisch bestimmt wird.

**22.** Verfahren nach Anspruch 19, **dadurch gekennzeichnet, dass** die Gebrauchslösung 0,02 bis 10 g/l, bevorzugter 0,05 bis 8 g/l, bevorzugter 0,1 bis 5 g/l, Wirkstoff enthält.

23. Verfahren nach einem der Ansprüche 19 bis 22, **dadurch gekennzeichnet, dass** die Lösung, deren UV-A-Tracer-Konzentration absorptionsspektralphotometrisch bestimmt werden soll, vor der absorptionsspektralphotometrischen Messung mit nichtionischem Tensid, insbesondere einem Alkylphenolethoxylat, versetzt wird.

24. Verfahren nach einem der Ansprüche 19 bis 23, **dadurch gekennzeichnet, dass** die Lösung, deren UV-A-Tracer-Konzentration absorptionsspektralphotometrisch bestimmt werden soll, vor der absorptionsspektralphotometrischen Messung durch Zugabe von Säure, Base oder Puffer auf einen gewünschten pH-Wert gebracht wird.

**Revendications**

1. Composition **caractérisée en ce qu'**elle comprend

   a) un ou plusieurs ingrédient(s) actif(s) microbicide(s) et
   b) un ou plusieurs traceur(s) UVA choisi(s) parmi la benzophénone et ses dérivés, l'acide anthranilique et ses dérivés,

   et éventuellement d'autres auxiliaires et/ou solvants/promoteurs de solubilité.

2. Composition selon la revendication 1, **caractérisée en ce que** l'ingrédient actif est choisi parmi un composé ammonium quaternaire, un dérivé de la guanidine, un dérivé du biguanide, un dérivé de la glycine, un aldéhyde et une alkylamine.

3. Composition selon la revendication 2, **caractérisée en ce que** le composé ammonium quaternaire est choisi parmi un chlorure d'alkylbenzyldiméthylammonium, un chlorure de dialkyldiméthylammonium, un éthosulfate d'alkyldiméthyléthyle, un propionate de dialkylméthyloxéthylammonium et le dichlorhydrate d'octénidine.

4. Composition selon la revendication 2, **caractérisée en ce que** le dérivé de la guanidine est choisi parmi le dichlorhydrate de chlorhexidine et le diacétate de cocopropylènediamineguanidinium.

5. Composition selon la revendication 2, **caractérisée en ce que** le dérivé de la glycine est un dérivé N-(3-aminopropyl) -N'-$C_{10-16}$-alkylglycine.

6. Composition selon la revendication 2, **caractérisée en ce que** le dérivé du biguanide est le polyhexaméthylènebiguanide.

7. Composition selon la revendication 2, **caractérisée en ce que** l'aldéhyde est choisi parmi le formaldéhyde, le succinaldéhyde, le glyoxal, le glutaraldéhyde et l'éthylhexanal.

8. Composition selon la revendication 2, **caractérisée en ce que** l'alkylamine est la N,N'-bis(3-aminopropyl)-dodécylamine.

9. Composition selon l'une quelconque des revendications 1 à 8, **caractérisée en ce que** le traceur UVA est choisi parmi la 2-hydroxy-4-méthoxybenzophénone, l'anthranilate de menthyle, l'anthranilate de méthyle, l'anthranilate d'éthyle et leurs dérivés, en particulier l'anthranilate de menthyle et la 2-hydroxy-4-méthoxybenzophénone.

10. Composition selon l'une quelconque des revendications 1 à 9, **caractérisée en ce que** l'auxiliaire est choisi parmi des tensioactifs, des agents complexants, des agents augmentant le pH, des inhibiteurs de corrosion, des colorants et des parfums, et leurs mélanges.

11. Composition selon l'une quelconque des revendications 1 à 10, **caractérisée en ce que** le solvant/le promoteur de solubilité est choisi parmi l'eau, des alcools aliphatiques et aromatiques, des glycols, des mono- et diéthers de glycol, des polyols et des mono-, des di- et des polyéthers de polyol, en particulier l'eau, l'isopropanol et l'éthylèneglycol, et leurs mélanges.

12. Composition selon l'une quelconque des revendications 1 à 11, **caractérisée en ce que**, dans la plage de la longueur d'onde mesurée, le coefficient d'absorption molaire $\varepsilon$ du traceur UVA est dans la plage de 500 à 200 000 l . mol$^{-1}$ . cm$^{-1}$, de préférence de 3000 à 30 000 l . mol$^{-1}$ . cm$^{-1}$, plus préférablement de 8000 à 15 000 l . mol$^{-1}$ . cm$^{-1}$, en particulier d'environ 10 000 l . mol$^{-1}$ . cm$^{-1}$.

13. Composition selon l'une quelconque des revendications 1 à 13, **caractérisée en ce qu'**elle comprend un aldéhyde et/ou une cétone, et le traceur UVA est la benzophénone ou un dérivé, en particulier la 2-hydroxy-4-méthoxybenzophénone.

14. Composition selon l'une quelconque des revendications 1 à 13, **caractérisée en ce que** le rapport pondéral (pds./pds.) entre l'ingrédient actif et le traceur UVA est dans la plage de 2000:1 à 1:300, de préférence de 850:1 à 1:80 et plus préférablement de 170:1 à 1:50.

15. Composition selon l'une quelconque des revendications 1 à 14 sous la forme d'un concentré, **caractérisée en ce que** le concentré comprend de 0,05 % en poids à 90 % en poids, de préférence de 0,1 % en poids à 80 % en poids, plus préférablement de 4 % en poids à 70 % en poids d'ingrédient actif.

16. Concentré selon la revendication 15, **caractérisé en ce qu'**il comprend de 0,05 à 15 % en poids, de préférence de 0,1 à 8 % en poids, plus préférablement de 0,5 à 7 % en poids de traceur UVA.

17. Utilisation d'une composition selon l'une quelconque des revendications 1 à 16 pour la désinfection et/ou le nettoyage, en particulier la désinfection de surfaces, le nettoyage de surfaces et la désinfection d'instruments.

18. Utilisation d'une composition selon l'une quelconque des revendications 1 à 16 pour la détermination de concentrations en ingrédient actif dans une solution ou sur des surfaces, en particulier pour déterminer le degré de dilution.

19. Procédé de détermination de la concentration en ingrédients actifs microbicides dans une composition qui est une solution de travail obtenue par dilution d'une partie en volume d'un concentré selon la revendication 15 ou 16 avec de 5 à 10 000 parties en volume, de préférence de 50 à 5000 parties en volume, plus préférablement de 100 à 2500 parties en volume d'eau, **caractérisé en ce que** la concentration en traceur UVA est déterminée au moyen de la spectrophotométrie d'absorption.

20. Procédé selon la revendication 19, **caractérisé en ce que** la composition comprenant un ingrédient actif et un traceur UVA est rincée ou éliminée par rinçage en utilisant une solution de rinçage et la concentration en traceur UVA restant dans la solution de rinçage après le rinçage ou l'élimination par rinçage est déterminée au moyen de la spectrophotométrie d'absorption.

21. Procédé selon la revendication 20, **caractérisé** en qu'une composition comprenant un ingrédient actif et un traceur UVA est appliquée sur une surface, la composition appliquée est rincée de la surface en utilisant une solution de rinçage et la concentration en traceur UVA dans la solution de rinçage est déterminée au moyen de la spectrophotométrie d'absorption.

22. Procédé selon la revendication 19, **caractérisé en ce que** la solution de travail comprend de 0,02 à 10 g/l, plus préférablement de 0,05 à 8 g/l, plus préférablement de 0,1 à 5 g/l d'ingrédient actif.

23. Procédé selon l'une quelconque des revendications 19 à 22, **caractérisé en ce que** la solution dont la concentration en traceur UVA est destinée à être déterminée au moyen de la spectrophotométrie d'absorption est traitée, avant la mesure de spectrophotométrie d'absorption avec un tensioactif non ionique, en particulier un éthoxylate d'alkylphénol.

24. Procédé selon l'une quelconque des revendications 19 à 23, **caractérisé en ce que** la solution dont la concentration en traceur UVA est destinée à être déterminée au moyen de la spectrophotométrie d'absorption est transformée, avant la mesure de spectrophotométrie d'absorption, pour donner lieu à un pH souhaité par addition d'un acide, d'une base ou d'un tampon.